# EUROPÄISCHE PATENTSCHRIFT

(11) **EP 2 351 541 B1**
(45) Veröffentlichungstag und Bekanntmachung des Hinweises auf die Patenterteilung: **07.06.2017**
(21) Anmeldenummer: 11162971.3
(22) Anmeldetag: 12.01.2006
(51) Int. Cl.: A61F 2/24

(54) **Katheter für die transvaskuläre Implantation von Herzklappenprothesen**
Catheter for transvascular implantation of heart valve prosthetics
Cathéter pour l'implantation transvasculaire de prothèses de valvules cardiaques

(30) Priorität: 20.01.2005 DE 102005003632
(43) Veröffentlichungstag der Anmeldung: 03.08.2011
(62) Teilanmeldung aus: 06705795.0
(73) Patentinhaber: JenaValve Technology, Inc., Irvine, CA 92618 (US)
(72) Erfinder: Ottma, Rüdiger, 99441, Grossschwabhausen (DE); Moszner, Robert, 07639, Bad-Klosterlausnitz (DE); Damm, Christoph, 07743, Jena (DE); Figulla, Hans-Reiner, 07749, Jena (DE); Ferrari, Markus, 07747, Jena (DE)
(74) Vertreter: Trinks, Ole

(56) Entgegenhaltungen:
- EP-A1- 1 369 098
- WO-A1-00/02503
- WO-A1-2004/019825

## Beschreibung

Die Erfindung betrifft Katheter für die transvaskuläre Implantation von Herzklappenprothesen mit selbstexpandierenden Verankerungsstützen, mit denen eine minimal-invasive Implantation von Herzklappenprothesen möglich ist.

Häufig und mit steigender Anzahl müssen an Patienten Herzklappenprothesen implantiert werden, wobei sowohl künstliche, wie auch biologische Implantate für Herzklappenprothesen eingesetzt werden.

Bisher werden solche Operationen so durchgeführt, dass am narkotisierten Patienten eine Herz-Lungen-Maschine eingesetzt werden muss. Dabei handelt es sich um einen kostenintensiven operativen Eingriff, bei dem die jeweiligen Patienten einer hohen psychisehen und physischen Belastung ausgesetzt sind. Das Letalitätsrisiko sollte unter 3 % gehalten sein. Mit ansteigendem Alter der jeweiligen Patienten sowie auch durch fortgeschrittene Beeinträchtigung der jeweiligen Herzklappen kommt es dazu, dass Patienten, die eigentlich behandlungsbedürftig sind, inoperabel sind. Da bei diesen Patienten ein operativer Klappenersatz nicht möglich ist, leiden sie unter verringerter Lebensqualität und weisen eine erheblich reduzierte Lebenserwartung auf. Das Risiko des Eingriffs wäre extrem hoch.

Dieser Sachverhalt trifft auch auf Operationen zu, bei denen Herzklappenprothesen mit Verankerungssystemen mittels so genannter Ballon-Katheter implantiert werden.

Bei einer solchen Vorgehensweise können Fehlpositionierungen auftreten, die für den Patienten erhebliche Folgen aufweisen können, was bis zum Tode des jeweiligen Patienten führen kann.

So wurde in der jüngeren Vergangenheit versucht, Herzklappenprothesen mittels minimal-invasiver Eingriffe zu implantieren, wobei solche Prothesen mit Verankerungsstützen über die Aorta eines Patienten ein und durch die Aorta bis zum Herzen geführt werden. Bei Erreichung des Implantationsortes am Herzen wurde eine Selbstexpansion solcher Verankerungsstützen, an denen eine Herzklappenprothese befestigt ist, initiiert, die zu einer sicheren Fixierung und exakten Positionierung der Herzklappenprothese führen sollte. Solche Verankerungsstützen wurden aus Formgedächtnislegierungen, wie beispielsweise "Nitinol" vorab hergestellt und dabei wurde die Legierung so ausgewählt, dass ihre Sprungtemperatur bei ca. 37 °C liegt und nach Erreichen der Sprungtemperatur die Selbstexpansion auslösbar ist.

Durch eine solche Expansion spannt sich die Verankerungerungsstütze so auf, dass sie sich an der Aortenwandung anlegen kann und gegebenenfalls mittels zusätzlicher Widerhakenelemente dort sicher fixiert werden kann. Gleichzeitig wird die Herzklappenprothese aufgefaltet, so dass sie ihre Funktion übernehmen kann.

Eine solche Verankerungsstütze mit Herzklappenprothese ist beispielsweise in WO 2004/019825 A1 beschrieben.

An einer solchen Verankerungsstütze sind proximalseitig Stützbügel ausgebildet, die in Taschen der Herzklappe eines Patienten eingeführt werden können, so dass die Verankerungsstütze bei einem chirurgischen Eingriff mittels dieser Stützbügel sehr genau positioniert werden kann. An dieser Verankerungsstütze sollen zusätzlich so genannte Kommissurenbügel ausgebildet sein, die gemeinsam mit den Stützbügeln Teile der alten Herzklappe eines Patienten nach erfolgter Entfaltung der Verankerungsstütze einklemmen, so dass infolge dieser Klemmwirkung die Verankerungsstütze sicher positioniert und fixiert werden kann.

Die Stütz- und Kommissurenbügel dieser bekannten Verankerungsstütze sollen dabei so angeordnet und auch dimensioniert sein, dass eine sequentielle Selbstexpansion erfolgen kann. Dies bedeutet, dass die Verankerungsstütze bei der Implantation innerhalb einer Kartusche aufgenommen ist. Sie wird dann über einen Katheter durch Aorta bis hin zum erkrankten Herzen geführt. Nach Erreichung des Implantationsortes soll die Kartusche so manipuliert werden, dass eine Freigäbe der Stützbügel für ihre Selbstexpansion erfolgen kann. Nachfolgend wird die Kartusche mit Verankerungsstütze so bewegt und ausgerichtet, dass die Stützbügel in die Taschen der Herklappen des jeweiligen Patienten eingeführt werden. Dadurch kann eine exakte Positionierung erreicht werden.

Im Anschluss daran wird die jeweilige Kartusche wiederum weiter manipuliert, so dass auch die Kommissurenbügel freigegeben werden und selbst expandieren können. Dabei wird die alte Herzklappe zwischen Stütz- und Kommissurenbügel eingeklemmt und die Herzklappenprothese in ihre entfaltete Funktionsstellung aufgespannt.

Nach erfolgter Implantation von Verankerungsstütze mit Herzklappenprothese kann dann der Katheter mit der Kartusche wieder über die Aorta aus dem Körper des Patienten entfernt werden.

Obwohl, wie bereits angesprochen, mit Hilfe der an der Verankerungsstütze ausgebildeten Stützbügel eine deutlich vereinfachte und verbesserte Positionierung der zu implantierenden Herzklappenprothese erreicht werden kann, besteht die Möglichkeit, dass es zu Fehlimplantationen kommen kann und die Herzklappenprothese nicht oder nur unbefriedigend funktionsfähig ist. Eine Entfernung einer so nicht oder unbefriedigenden funktionsfähigen Herzklappenprothese ist dann in einigen Fällen nicht mehr möglich bzw. es besteht für den jeweiligen Patienten ein erhöhtes Mortalitätsrisiko.

Ein weiteres Problem bei solchen chirurgischen Eingriffen stellt dabei auch der Aortenbogen am menschlichen Körper dar, der beim Einführen über die Aorta durchdrungen werden muss. Dabei muss bei der Bewegung von Kartusche und dem jeweiligen Katheter eine Richtungsänderung von ca. 180° mit einem relativ geringen Radius von ca. 50 mm durchgeführt werden, ohne dass es zu einer Verletzung der Gefäßwand kommt.

Die Druckschrift EP 1 369 098 A1 betrifft ein Zufuhrsystem zum Implantieren eines Stents, wobei das Zufuhrsystem eine herausziehbare Primärhülse aufweist, welche den Stent in einer ersten Konfiguration mit eingeschränktem Durchmesser enthält. Des Weiteren ist ein äußerer Schlauch innerhalb der herausziehbaren Primärhülse und innerhalb des Stents vorgesehen. Darüber hinaus ist ein innerer Schlauch innerhalb des äußeren Schlauches vorgesehen, wobei der innere Schlauch und der äußere Schlauch sich beide axial relativ zur herausziehbaren Primärhülse und zueinander bewegen können.

Die Druckschrift WO 2004/019825 offenbart einen Katheter gemäß dem Oberbegriff des Anspruchs 1.

Es ist Aufgabe der Erfindung, das Patientenrisiko bei einer Implantation von Herzklappenprothesen zu reduzieren.

Erfindungsgemäß wird diese Aufgabe mit einem Katheter, welcher die Merkmale des Anspruchs 1 aufweist, gelöst. Vorteilhafte Ausgestaltungsformen und Weiterbildungen der Erfindung können mit den in den untergeordneten Ansprüchen bezeichneten Merkmalen erreicht werden.

In Verbindung mit dem erfindungsgemäßen Katheter kann in bevorzugter Form eine Herzklappenprothese mit selbstexpandierender Verankerungsstütze, wie sie aus WO 2004/019825 A1 bekannt ist und auf deren Offenbarungsgehalt hiermit Bezug genommen wird, eingesetzt werden.

Dabei sind Verankerungsstütze mit der daran befestigten Herzklappenprothese während der Implantation innerhalb einer Kartuscheneinheit temporär in zusammengefalteter Form aufgenommen.

Eine so vorbereitete Kartuscheneinheit kann an einem Führungssystem proximalseitig lösbar befestigt werden. Kartuscheneinheit und Führungssystem sind mit ihrem Aussendurchmesser insoweit minimiert, dass sie ohne weiteres durch eine Aorta eines zu operierenden Patienten hindurchgeführt werden können, wobei der gesamte innerhalb der Aorta zur Verfügung stehende freie Querschnitt nicht vollständig ausgefüllt sein sollte.

Das Führungssystem hat dabei eine ausreichend große Länge, um die Kartuscheneinheit mit dem Führungssystem über eine Einführung an der Leiste eines Patienten durch die Aorta bis hin zum Herzen des Patienten führen zu können.

Am Führungssystem ist ein flexibler, biegbarer Bereich vorhanden, mit dem ein Biegeradius und Biegewinkel realisiert werden kann, der dem Aortenbogen eines Patienten folgen und berücksichtigen kann.

Durch das im Inneren hohle Führungssystem sind Betätigungselemente zur Kartuscheneinheit geführt. Mit diesem Betätigungselement können Teile der Kartuscheneinheit in gezielter Form manipuliert und bewegt werden. So kann mit den Betätigungselementen eine radiale oder auch laterale Bewegung von Teilen der Kartuscheneinheit erreicht werden. Mit Hilfe dieser gezielten Bewegung von Teilen der Kartuscheneinheit kann dann eine sequentielle Freigabe von Teilen der Verankerungsstütze erreicht werden, so dass die Implantation mit Verankerung, wie im einleitenden Teil der Beschreibung und in WO 2004/019825 A1 beschrieben, erfolgen kann.

So kann beispielsweise eine Freigabe von Stützbügeln einer Verankerungsstütze durch eine Verdrehung oder eine laterale Bewegung in proximaler oder distaler Richtung eines Teiles der Kartuscheneinheit erreicht werden und dabei aber andere Teile, wie beispielsweise Kommissurenbügel noch innerhalb der Kartuscheneinheit in zusammengefalteter Form gehalten bleiben, die dann nachfolgend durch eine entsprechende Bewegung eines weiteren Teiles der Kartuscheneinheit oder einer weiterführenden Bewegung des gleichen Teiles der Kartuscheneinheit das vorab die Stützbügel noch im zusammengefalteter Form innerhalb der Kartuscheneinheit gehalten hat, für eine Expansion freigegeben werden können.

Das Aufspannen der Herzklappenprothese, die an der Verankerungsstütze, beispielsweise durch Vernähen befestigt worden ist, wird dann gleichzeitig mit der Expansion der jeweiligen Bügel der Verankerungsstütze, an denen die Herzklappenprothese befestigt ist, erreicht.

In bevorzugter Ausführungsform sind neben den Betätigungselementen für Teile der Kartuscheneinheit weitere Betätigungselemente durch das innen hohle Führungssystem geführt, die am biegbaren Bereich für eine gezielte Beeinflussung seiner Krümmung angreifen.

Insbesondere durch die Auslösung von Zugkräften über die Betätigungselemente kann eine gezielte Krümmung des biegbaren Bereiches während der Implantation beim Durchdringen des Aortenbogens erreicht werden. So können als Betätigungselemente Zugseile oder Zugdrähte durch das innen hohle Führungssystem bis an den proximalen Rand des biegbaren Bereiches geführt und dort am Führungssystem befestigt sein, wobei die Kraftangriffspunkte von zwei solcher Betätigungselemente dann sich diametral gegenüberliegend angeordnet sein sollten und außerdem eine Anordnung, um jeweils 90° zur Biegeachse, um die der biegbare Bereich gekrümmt werden soll, gewählt werden.

So kann eine gezielte Beeinflussung der Krümmung des biegbaren Bereiches durch Ausübung einer Zugkraft über eines der vorhandenen Betätigungselemente erreicht werden, wenn das Führungssystem mit dem biegbaren Bereich durch den Aortenbogen geschoben bzw. nach erfolgter Implantation aus diesem herausgezogen wird.

Der biegbare Bereich eines Führungssystems kann dabei in Form einer Gliederkette ausgebildet sein, in dem die einzelnen Glieder über Einzelgelenke miteinander verbunden sind. Die Einzelgelenke greifen dabei formschlüssig in jeweils benachbarte Glieder ein. Sie sind dabei so ausgebildet, dass eine Krümmung des biegbaren Bereichs um mehr als 180° mit einem Biegeradius, der gewährleistet, dass mindestens der Radius des Aortenbogens erreichbar ist, eingehalten werden können.

Die Einzelgelenke an den einzelnen Gliedern einer Gliederkette sollten dabei ebenfalls sich paarweise diametral gegenüberliegend an den einzelnen Gliedern ausgebildet und parallel zur Drehachse des biegbaren Bereiches angeordnet sein.

Das an einem erfindungsgemäßen Katheter eingesetzte Führungssystem sollte vorteilhaft auch so ausgebildet sein, dass ein flüssiges Kühlmittel bzw. Pharmaka durch das innen hohle Führungssystem bis zur Kartuscheneinheit geführt werden kann. Mit Hilfe eines solchen flüssigen Kühlmittels, beispielsweise einer Kochsalzlösung kann die Verankerungsstütze unterhalb der Sprungtemperatur, der Formgedächtnislegierung gehalten werden. Außerdem kann dadurch auch vermieden werden, dass Körperflüssigkeit in das Innere des Führungssystems eintreten kann, wobei ein entsprechender Flüssigkeitsdruck eingehalten werden sollte, der dem Eindringen von Körperflüssigkeit oder in Körperflüssigkeit enthaltenden weiteren Bestandteilen einen ausreichend großen Widerstand leistet.

Mit entsprechender Einführung des flüssigen Kühlmittels kann aber auch verhindert werden, dass Gas, beispielsweise Luft, in die Aorta und das Blut gelangen kann.

Hierzu sollte das gesamte Führungssystem möglichst flüssigkeitsdicht ausgebildet sein. Dementsprechend kann ein flexibler, biegbarer Bereich, der in Form einer Gliederkette ausgebildet ist, mit Hilfe eines Kunststoffschlauches von außen flüssigkeitsdicht abgedichtet sein.

Die Teile der Kartuscheneinheit, die für eine Freigabe von Bügeln der Verankerungsstütze gezielt bewegt werden können, sind bevorzugt als hülsenförmige Elemente ausgebildet, deren Innen- und Außendurchmesser so aufeinander abgestimmt sind, dass sie teleskopförmig ineinander greifen können, wobei mindestens zwei der hülsenförmigen Elemente so aufeinander abgestimmte Innen-und Außendurchmesser aufweisen, dass zwischen ihnen eine zusammengefaltete Verankerungsstütze mit Herzklappenprothese aufgenommen ist und in zusammengefalteter Form gehalten werden kann.

Beim Einführen des Katheters sollte die Kartuscheneinheit möglichst vollständig geschlossen sein und für ein erleichtertes Einführen durch die Aorta proximalseitig eine Spitze aufweisen, die wiederum besonders bevorzugt aus einem flexiblen Werkstoff, beispielsweise Silikon gebildet ist.

Bei Erreichen der Kartuscheneinheit des Herzens des jeweiligen Patienten kann dann die entsprechende Manipulation, also die Bewegung von Teilen/hülsenförmigen Elementen der Kartuscheneinheit so durchgeführt werden, dass die unterschiedlichen Bügel der Verankerungsstütze sequentiell freigegeben werden und gleichzeitig die daran befestigte Herzklappenprothese aufgespannt wird.

Dabei bleibt aber die Verankerungsstütze noch an der Kartuscheneinheit fixiert gehalten. Hierfür sind an einem hülsenförmigen Element der Kartuscheneinheit Verankerungselemente ausgebildet, die distal, beispielsweise an dort an der Verankerungsstütze ausgebildeten Ösen angreifen. Diese Verankerungselemente sind in dieser Position gemeinsam mit dem distalen Teil der Verankerungsstütze von einem hülsenförmigen Element der Kartuscheneinheit auch überdeckt, so dass der distale Teil der Verankerungsstütze noch zusammengefaltet gehalten wird.

In dieser Stellung ist eine Überprüfung der Funktion der bereits entfalteten Herzklappenprothese möglich. Nach erfolgtem Funktionsnachweis der Herzklappenprothese kann dann eine weitere Manipulation, durch entsprechende Bewegung des vorab die Verankerungselemente mit distalem Teil der Verankerungsstütze überdeckenden hülsenförmigen Elementes, erfolgen, die zu einer vollständigen Freigabe auch des distalen Teiles der Verankerungsstütze und dementsprechend zur vollständigen Entfaltung führt.

Wird bei der Prüfung festgestellt, dass die implantierte Herzklappenprothese ihre Funktion nicht oder nur unzulänglich erfüllen kann, besteht in besonders vorteilhafter Weise die Möglichkeit durch entsprechend entgegengesetzt gerichtete Bewegung der Teile/hülsenförmigen Elemente die Verankerungsstütze mit Herzklappenprothese wieder in die Kartuscheneinheit zurückzubewegen und sämtliche Teile, also das gesamte Katheter durch die Aorta aus dem Körper des Patienten wieder zu entfernen, so dass das Operationsrisiko erheblich reduziert und nachfolgend ein weiterer Versuch einer Implantation am selben Patienten durchgeführt werden kann.

In einer vorteilhaften Ausführungsform des erfindungsgemäßen Katheters kann auch ein Führungsdraht durch den gesamten Katheter hindurchgeführt sein. Solche Führungsdrähte werden bei ähnlichen Operationen bereits eingesetzt, wobei sie vor dem Einführen des Katheters durch die Aorta des Patienten bis hinter das Herz geführt werden. Der Katheter kann dann über Kartuscheneinheit und Führungssystem in den Führungsdraht eingefädelt und entlang dessen in die Aorta bis hin zum Herzen des Patienten eingeschoben werden.

Für eine Überwachung des Einführvorganges des Katheters und auch die Manipulation des biegbaren Bereiches, insbesondere im Aortenbogen ist es vorteilhaft am Führungssystem und/oder der Kartuscheneinheit Markierungselemente vorzusehen, die aus einem Werkstoff gebildet sind, der Röntgenstrahlung absorbiert, so dass sich während der Operation die jeweilige Position an einem Röntgenbild erkennen lässt.

An einem erfindungsgemäßen Katheter kann aber auch ein Schirmfilter eingesetzt werden, mit dem das Eindringen von Partikeln in die Blutbahn des jeweiligen Patienten verhindert werden kann. Ein solches Schirmfilter kann am Führungssystem so befestigt sein, dass es dieses radial vollständig umgreift. Dabei sollte es elastisch vorgespannt sein, so dass es sich an die Gefäßwand an die Aorta anlegt und ein partikeldichter Verschluss gewährleistet werden kann.

Der erfindungsgemäße Katheter kann außerdem zusätzlich mit einem herkömmlichen Ballon ausgebildet sein, der im Inneren des Führungssystems oder der Kartuscheneinheit angeordnet und dort mitgeführt bzw. auch durch das Innere des Führungssystems bis hin zur expandierenden Verankerungsstütze geführt werden kann. Mit einem solchen Ballon, der beispielsweise durch eine unter erhöhtem Druck stehende Flüssigkeit in seinem Volumen entsprechend vergrößert werden kann, kann die Expansion der Verankerungsstütze noch unterstützt werden.

Die bereits angesprochenen, durch das Innere des Führungssystems geführten Betätigungselemente, die als Zug- und Druckmittel ausgebildet sein können, können vorteilhaft von einem Handhabungsteil aus manipuliert werden. Das Handhabungsteil kann als Handgriff ausgebildet sein, über den dann die Bewegung für das Einführen des Katheters vom jeweiligen Operateur ausgeübt werden kann.

An einem solchen Handhabungsteil sind dann weitere Bedienelemente vorhanden, über die die jeweilige Bewegung der Betätigungselemente ausgelöst werden kann. Dabei sollte die entsprechende Bewegung in möglichst dosierter Form, beispielsweise mit entsprechenden Übersetzungsverhältnissen erfolgen können und eine Begrenzung der jeweiligen Bewegung durch zumindest Endanschläge oder Rasteinstellungen begrenzt werden können. Dadurch lassen sich bestimmte maximale Wege oder Winkel einhalten, die für die sequentielle Expansion der Verankerungsstütze oder die gezielte Beeinflussung der Krümmung des biegbaren Bereiches berücksichtigt werden können. Dabei sollte eine möglichst feine Justierung der Endanschläge bzw. einzelner Raster möglich sein.

Sämtliche Teile eines erfindungsgemäßen Katheters, zumindest jedoch die die mit dem jeweiligen Patienten in unmittelbarem Kontakt treten bzw. auch in die Aorta eingeführt werden, sollten aus biokompatiblen Werkstoffen hergestellt worden sein, die vom jeweiligen Organismus vertragen werden können. Zusätzlich sollte eine Sterilisation möglich sein, wobei zumindest eines der gängigen Sterilisationsverfahren einsetzbar sein sollte.

Nachfolgend soll die Erfindung beispielhaft näher erläutert werden.

Dabei zeigen:
- Figuren 1 bis 4: ein Beispiel eines erfindungsgemäßen Katheters bei unterschiedlichen möglichen Phasen eines Implantationsvorganges in schematischer Form;
- Figur 5: ein Beispiel eines Katheters mit einem Handhabungsteil und
- Figur 6: das Handhabungsteil nach Figur 5 in einer Explosionsdarstellung.

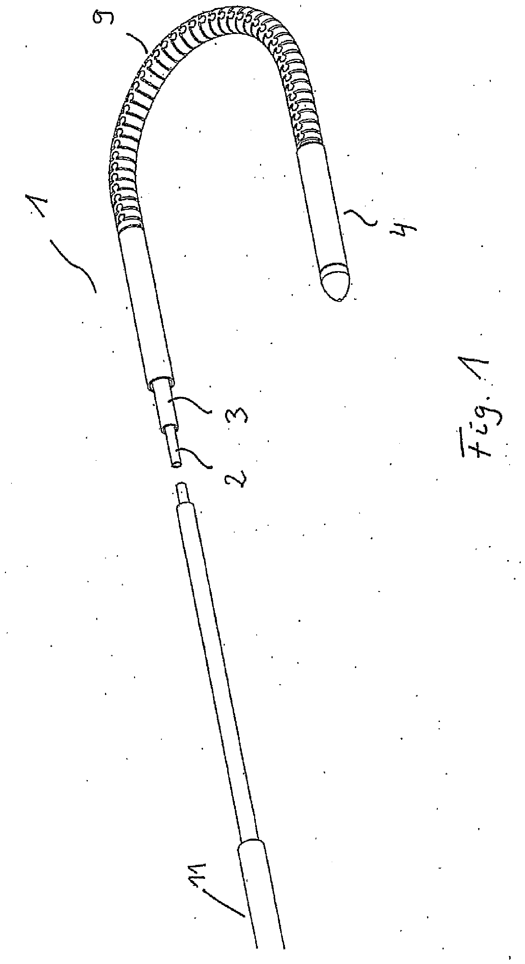

Mit den Figuren 1 bis 4 soll ein Beispiel für einen erfindungsgemäßen Katheter dargestellt und besser verständlich gemacht werden. Dabei sind in den einzelnen Abbildungen unterschiedliche Phasen, die während einer Implantation einer Verankerungsstütze 10 mit Herzklappenprothese durchlaufen werden können, dargestellt.

In Figur 1 ist das Beispiel eines erfindungsgemäßen Katheters, so gezeigt, dass in der Kartuscheneinheit 4, die noch vollständig geschlossen ist, eine Verankerungsstütze 10 mit Herzklappenprothese in nicht expandierter Form und entsprechend zusammengefaltet aufgenommen ist und so mittels des inneren hohlen Führungssystems 1 über einen geeigneten Zugang in die Aorta und durch diese zum jeweiligen Implantationsort am Herzen des Patienten hindurchgeführt werden kann.

An der Kartuscheneinheit 4 ist proximal eine flexible Spitze aus Silikon vorhanden, mit der das Einführen erleichtert und Verletzungsgefahren reduziert werden können.

Das Teil 5 der Kartuscheneinheit ist lösbar mit den anderen Teilen des Führungssystems 1, beispielsweise durch eine Verschraubung, mit diesem verbunden.

An die Kartuscheneinheit 4 schließt sich ein biegbarer Bereich 9 an, der so ausgebildet und dimensioniert ist, dass eine problemlose Bewegung durch den Aortenbogen eines Patienten gewährleistet werden kann.

Auf Möglichkeiten zur Ausbildung eines solchen biegbaren Bereiches 9 soll später noch zurückgekommen werden.

Im Übrigen sind weitere Teile des innen hohlen Führungssystems 1 dargestellt, wobei in den Figuren 1 bis 4 zwei Betätigungselemente 2 und 3 durch das Führungssystem 1 bis zur Kartuscheneinheit 4 geführt sind, dabei ist das Betätigungselement 2, hier durch das ebenfalls innen hohle Betätigungselement 3 hindurch, bis zur Kartuscheneinheit 4 geführt.

Die Betätigungselemente 2 und 3 sind hier als Druckfederstränge, die bevorzugt mittels Zugdrähten verstärkt sind, ausgeführt. Durch solche Zugdrähte kann die Sicherheit bei der Entfernung des Katheters aus dem Körper des Patienten nach erfolgter Operation erhöht werden.

Links sind weitere Teile 11 des Führungssystems 1 dargestellt, die beispielsweise als mehr oder weniger flexible hülsenförmige Teile ausgebildet sein können, wobei jedoch gesichert sein soll, dass sie für das Einführen in die Aorta und das wieder Herausführen aus der Aorta eine ausreichend hohe Druck- und Zugfestigkeit aufweisen. Hier können entsprechend steife Kunststoffschläuche, beispielsweise PTFE-Schläuche oder PTFE-basierte Schläuche eingesetzt werden, da sie für den Organismus ausreichend verträglich und auch sterilisierbar sind.

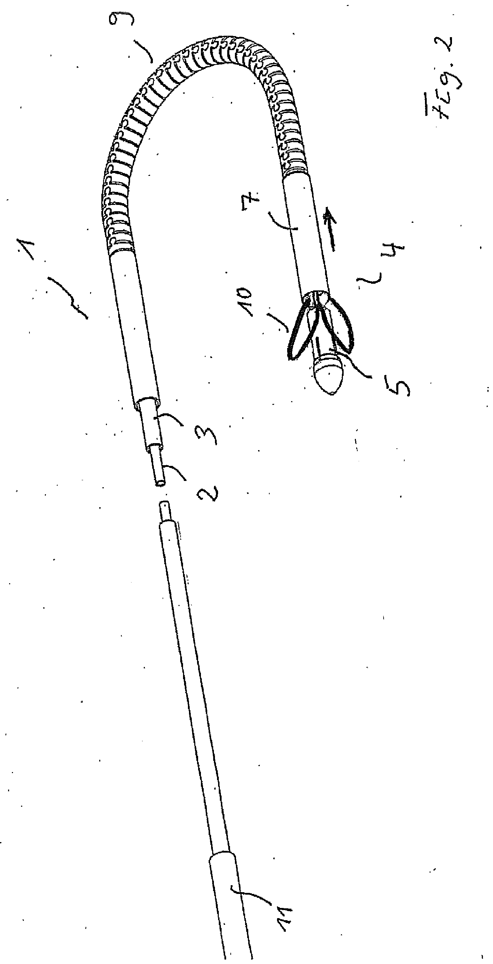

In Figur 2 ist dargestellt, wie in einem ersten Operationsstadium, nach Erreichen des Implantationsortes am Herzen des jeweiligen Patienten verfahren werden kann. Durch eine distale Bewegung eines der Betätigungselemente 2 und/oder 3 kann das Teil/hülsenförmige Element 7 der Kartuscheneinheit 4 distal zurückgezogen werden, so dass einige Bügel der Verankerungsstütze 10, beispielsweise und bevorzugt die Stützbügel, wie sie bei der aus WO 2004/019825 A1 bekannten Herzklappenprothese vorhanden sein sollen, expandieren und radial nach außen aufspannen.

So kann das gesamte Katheter mit Führungssystem 1 und der Kartuscheneinheit 4 proximal verschoben und so diese Bügel (Stützbügel) in die Taschen der alten Herzklappe des Patienten eingeführt werden. Verspürt der Operateur dann einen merklichen Widerstand, dürfte der Einführvorgang der Stützbügel, der Verankerungsstütze 10 in die Taschen der alten Herzklappe erfolgt sein.

Das Teil/hülsenförmige Element 5 der Kartuscheneinheit 4 kann dann weiter distal nach vorn bewegt werden, so dass auch weitere Bügel der Verankerungsstütze für eine Selbstexpansion freigegeben werden und die Herzklappenprothese aufgespannt wird.

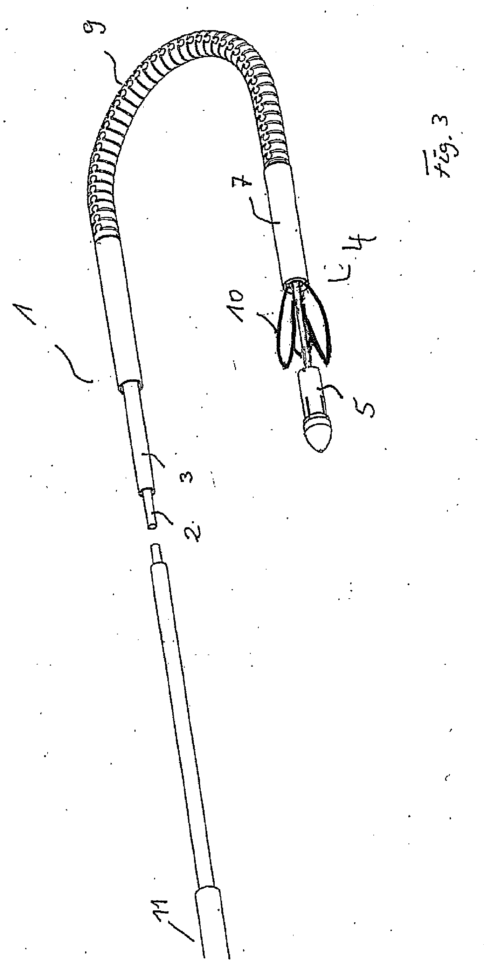

Eine Vorstufe hiervon ist in Figur 3 dargestellt, wobei hier noch keine vollständige Entfaltung einer Herzklappenprothese erreicht worden ist und auch noch keine vollständige Verankerung der Verankerungsstütze 10 ermöglicht wurde.

Mit Figur 3 wird außerdem deutlich, dass ein distaler Teil der Verankerungsstütze 10 noch innerhalb der Kartuscheneinheit 4, unterhalb des Teiles/hülsenförmigen Elementes 7 in der Kartuscheneinheit 4 aufgenommen ist. Dies bleibt solange der Fall, bis die Entfaltung und Positionierung der Herzklappenprothese in soweit erfolgt ist, dass diese auf ihre Funktionalität überprüft werden konnte.

Wird bei einer solchen Überprüfung eine Fehlfunktion oder Fehlpositionierung erkannt, kann das Teil/hülsenförmige Element 7 wieder über eines der beiden Betätigungselemente 2 oder 3 proximal verschoben werden, so dass die Verankerungsstütze 10 mit Herzklappenprothese zumindest teilweise wieder in die Kartuscheneinheit 4 aufgenommen wird und dann das gesamte Katheter aus dem Patienten durch Herausziehen aus der Aorta entfernt werden kann, ohne dass es zu Verletzungen der Gefäßwand kommt.

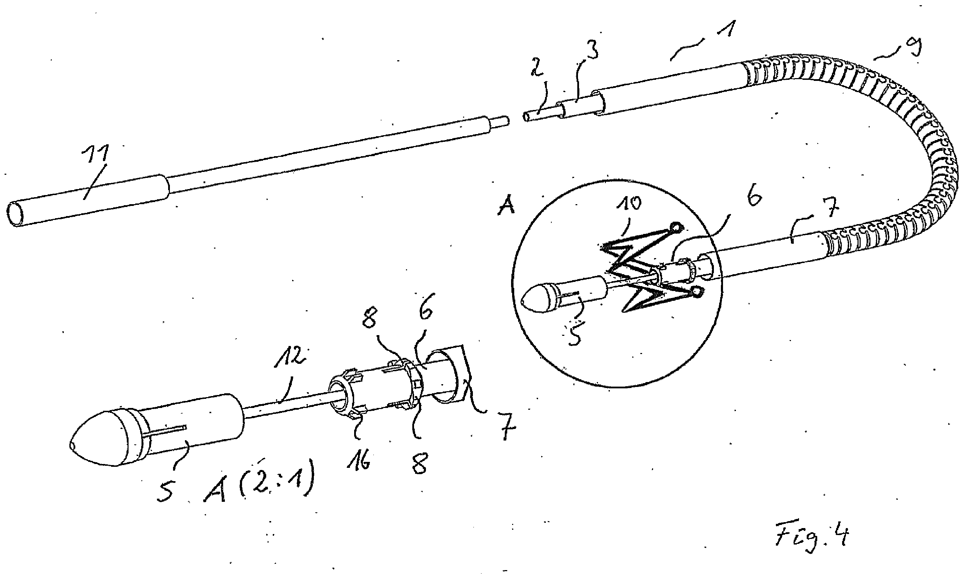

Ergibt sich bei der Funktionsprobe der Herzklappenprothese, dass diese ihre Funktion zumindest in einem ausreichenden Maße erfüllen kann, kann, wie in Figur 4 gezeigt, das Teil/hülsenförmige Element 7 weiter distal zurück und/oder ein weiteres Teil/hülsenförmiges Element 6 der Kartuscheneinheit 4 in proximale Richtung verschoben werden, so dass auch der distale Teil der Verankerungsstütze 10 vollständig frei gegeben und vollständig expandieren kann.

In Figur 4 wird außerdem deutlich, dass an distalen Endbereichen der Verankerungsstütze 10 Ösen oder andere geeignete Elemente ausgebildet worden sind, die vorab in Verankerungselemente 8, die am Teil/hülsenförmigen Element 6 ausgebildet sind, eingegriffen hatten. Über diese Ösen mit den Verankerungselementen 8 kann gesichert werden, dass bei festgestellter Fehl- oder Falschimplantation einer Verankerungsstütze 10 mit Herzklappenprothese ein sicheres Zurück- und Herausziehen, bei Mitführung von Verankerungsstütze 10 und Herzklappenprothese aus dem Körper des Patienten erreichbar ist.

Mittels der Verankerungselemente 8 und gegebenenfalls auch weiteren Führungselementen 16, die am Teil/hülsenförmigen Element 6, der Kartuscheneinheit 4 ausgebildet sein können, besteht auch die Möglichkeit, eine radiale Verdrehung vorzunehmen, um die Bügel einer Verankerungsstütze 10 auch in einer exakten geeigneten Winkellage, beispielsweise in die Taschen einer alten Herzklappenprothese einführen zu können, wobei während der Implantation das gesamte Katheter vom Operateur um seine Längsachse leicht verdreht werden kann.

Insbesondere in der Einzelheit A von Figur 4 ist auch eine Kanüle 12, die durch die Kartuscheneinheit 4 entlang ihrer Längsachse geführt ist, erkennbar. Über die Kanüle 4 kann beispielsweise der im allgemeinen Teil der Beschreibung erwähnte Führungsdraht durch die Kartuscheneinheit 4 geführt sein.

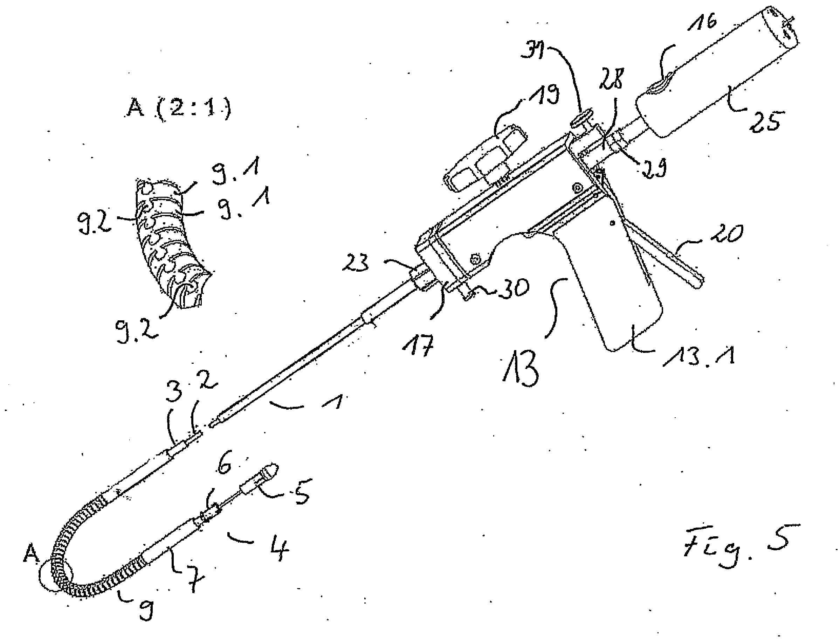

In Figur 5 ist ein Beispiel eines Katheters mit zusätzlichem Handhabungsteil 13, an dem weitere Bedienungselemente für die Manipulation vorhanden sind, dargestellt.

Dabei entspricht das bereits in den Figuren 1 bis 4 gezeigte Führungssystem 1 mit Kartuscheneinheit 4 auch dem hier gezeigten Beispiel.

Dabei ist mit der Einzelheit A jedoch eine mögliche Ausbildung des biegbaren Bereiches 9, als Gliederkette verdeutlicht worden.

Die einzelnen Glieder 9.1 sind dabei generell jeweils in gleicher Gestalt und Dimensionierung ausgebildet.

Die sich gegenüberliegenden Stirnseiten der einzelnen Glieder 9.1 sind dabei so gestaltet, dass sie Einzelgelenke 9.2 bilden, die jeweils formschlüssig in benachbarte einzelne Glieder 9.1 eingreifen und infolge von Spalten mit jeweils ausreichender Spaltbreite zwischen den einzelnen Gliedern 9.1 eine Biegung des biegbaren Bereiches, um die bereits angesprochenen mindestens 180°, bei einem Radius von ca. 50 mm gewährleisten.

Die Einzelgelenke 9.2 sind dabei durch entsprechende Ausformung der sich jeweils gegenüberliegenden Stirnseiten der einzelnen Glieder 9.1 gebildet worden, wobei eine entsprechende Aussparung an einer Stirnseite und eine entsprechende abgerundete komplementäre ausgebildete Erhebung an der diametral gegenüberliegenden Stirnseite der einzelnen Glieder 9.1 die Einzelgelenke 9.2 an jeweils benachbarten einzelnen Gliedern 9.1 bilden.

Der biegbare Bereich 9 kann in nicht dargestellter Form mit einem Kunststoffschlauch fluiddicht umschlossen sein.

In Figur 5 ist ferner verdeutlicht, wie ein Handhabungsteil 13 für das Einführen und die Manipulation eines erfindungsgemäßen Katheters ausgebildet sein kann.

Für das Einführen und Herausziehen des Katheters mit Führungssystem 1 und Kartuscheneinheit 4 ist ein Handgriff 13.1 vorhanden. Der Handgriff 13.1 des Handhabungsteils 13 dient dabei zum Steuern des eingeführten Katheters.

Im proximalen Teil des Handhabungsteiles 13 ist ein flüssigkeitsdichter Verschluss in Form einer Platte 17 vorhanden, an der die Möglichkeit für eine Anflanschung des Führungssystems 1 mittels einer Überwurfmutter 23 und hier nicht dargestellten Abdichtelementen vorhanden.

Des Weiteren ist ein standardisierter Lueranschluss 30 vorhanden, über den die Kühlflüssigkeit zugeführt werden kann.

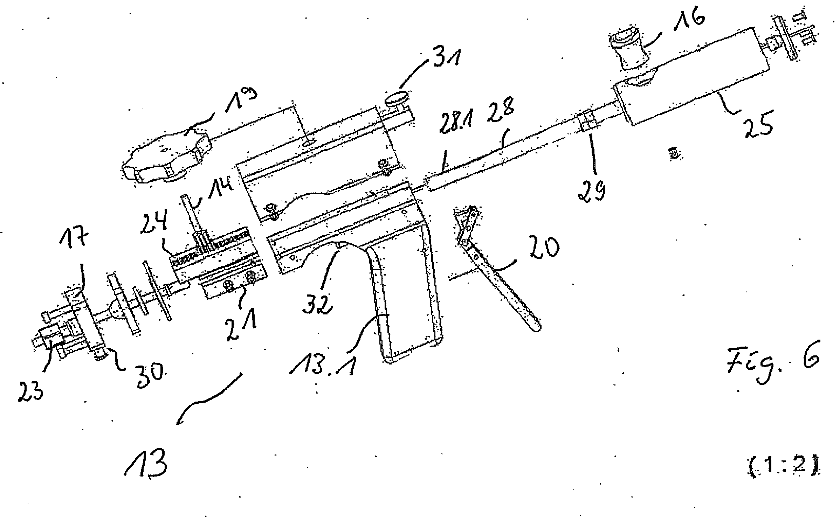

Mit dem Handgriff 19, der um eine Achse verdreht werden kann, kann die jeweilige Krümmung des biegbaren Bereiches 9 über Zugseile (nicht dargestellt) realisiert werden, wobei hierzu nähere Erläuterungen noch bei der Beschreibung der Figur 6 folgen.

Das gesamte Handhabungsteil 13 sollte möglichst flüssigkeits- und gegebenenfalls auch gasdicht gegenüber der Umwelt und dem Führungssystem 1 abgedichtet sein.

Mit dem am Handgriff 13.1 angreifenden Hebels 20 kann eine laterale Bewegung des Rohres 28 in proximaler Richtung erreicht werden, wodurch die entsprechende Bewegung und daraus resultierende Zug- bzw. Druckkraft auf eines der beiden Betätigungselemente 2 und/oder 3 übertragen werden kann, so dass eine Manipulation der einzelnen Teile/hülsenförmigen Elemente 5, 6 und/oder 7 der Kartuscheneinheit 4, wie beispielhaft bereits vorab beschrieben, in fein dosierter Form über pumpenförmige Bewegungen des Hebels 20 erreicht werden können.

Mit dem Drückergriff 25 kann in Verlängerung über den Federstrang, als Betätigungselement 2, die Position des Teiles 5 der Kartuscheneinheit 4 gegenüber dem hülsenförmigen Teil 6 der Kartuscheneinheit 4 mit dem Befestigungshaken, als Verankerungselemente 8 manipuliert werden. Der Drückergriff 25 wird mittels einer Druckfeder in einer gewindeförmigen Verzahnung 28.1 eines Rohres 28 eingerastet. In dieser Weise folgt der Drückergriff 25 der proximalen Bewegung des Rohres 28, das mit dem Teil 6 der Kartusche über den Federstrang, als Betätigungselement 3 verbunden ist.

Nach Erreichen eines Endanschlages zur ersten Entladestufe, kann durch Verdrehung des Drückergriffes 25 eine, im Sinne der Steigung des Gewindes 28.1 fein dosierte axiale Verschiebung des Teiles 5 der Kartuscheneinheit 4 gegenüber dem Teil 6 der Kartuscheneinheit 4 erzielt werden.

Für die Betätigung des Drückergriffes 25 kann dieser das Teil 5 der Kartuscheneinheit 4 hier ohne eine zusätzliche Feinjustierung bewegen.

Mit einer solchen Manipulation kann die Freigabe der Verankerungsstütze 10 (siehe Figur 3) erreicht werden und die Verankerungsstütze 10 ist in dieser Position noch zurückholbar.

Nach Freigabe des Anschlages 29 durch ein Stellglied 31, dass beispielsweise als Stellschraube ausgeführt ist, kann die Kartuscheneinheit 4 durch Betätigung des Hebelsystems 20, wie vorab beschrieben, weiter ausgefahren werden, bis die Halteösen der Verankerungsstütze 10 die Kartuscheneinheit 4 verlassen haben und so die Verankerungsstütze 10 durch ihre Expansionskräfte von den Verankerungselementen 8 abspringen können.

Die Elemente der Kartuscheneinheit 4 können stufenweise zurückgezogen werden. Dabei kann das Teil 5 der Kartuscheneinheit 4 durch Zurückziehen des Drückergriffes 25 (Drücker eingerastet) bis über das Teil 6 der Kartuscheneinheit 4 zurückgeholt werden.

Durch Betätigung eines Entriegelungsbolzens 32 kann durch weiteres Zurückziehen des Drückergriffes 25 auch das mit dem Rohr 28 verbundene Teil 6 der Kartuscheneinheit 4 in seine Ausgangsposition zurückgeholt werden, so dass dann die Kartuscheneinheit 4 wieder vollständig geschlossen ist. In diesem Zustand kann der Katheter wieder aus dem Körper des Patienten entfernt werden. Mit Figur 6 ist in Form einer Explosionsdarstellung das Handhabungsteil 13 für dieses Beispiel weiter verdeutlicht.

So ist erkennbar, dass durch Drehung des Handrades 19 über die Welle 14 eine Verschiebung von zwei parallel zueinander ausgerichteten Zahnstangen 24 erreicht werden kann. Dabei bewegt sich die eine Zahnstange 24 in proximaler Richtung, wenn sich die parallel dazu ausgerichtete Zahnstange 24 in distale Richtung bewegt.

An entsprechend an den Zahnstangen 24 angreifenden Klemmbacken 21 können hier ebenfalls nicht dargestellte Zugseile befestigt sein, die zum biegbaren Bereich 9 durch das innen hohle Führungssystem 1 geführt und bevorzugt in dessen proximalem Bereich befestigt sind.

So kann durch entsprechende Drehung des Handgriffes 19 auf mindestens eines der beiden Zugseile eine Zugkraft ausgeübt werden, die zur entsprechenden Krümmung des biegbaren Bereiches 9 in dosierter Form führt, so dass das Führungssystem 1 mit der Kartuscheneinheit 4 durch den Aortenbogen in definierter Form geführt werden kann.

In Figur 6 wird weiter deutlich, dass der mit dem Handgriff 13.1 verbundene Hebel 20 über eine feine Verzahnung 28.1 am Rohr 28 angreift und dieses dann entsprechend für die Manipulation, zur sequentiellen Freigabe der Verankerungsstütze 10 über die Betätigungselemente 2 und/oder 3 manipuliert werden kann.

## Patentansprüche

1. Katheter mit einem Handhabungsteil (13) und einer Kartuscheneinheit (4) zum transvaskulären Implantieren einer in zusammengefalteter Form in der Kartuscheneinheit (4) des Katheters aufgenommenen selbstexpandierenden Verankerungsstütze (10) mit einer daran befestigten Herzklappenprothese, wobei die Kartuscheneinheit (4) ein erstes hülsenförmiges Element (5) und ein zweites hülsenförmiges Element (7) aufweist,
**dadurch gekennzeichnet, dass**
die Kartuscheneinheit (4) ferner ein weiteres hülsenförmiges Element (6) aufweist, wobei an dem weiteren hülsenförmigen Element (6) der Kartuscheneinheit (4) Verankerungselemente (8) ausgebildet sind, in welchem an distalen Endbereichen der Verankerungsstütze (10) ausgebildete Ösen oder andere geeignete Elemente vorab in Eingriff gebracht sind; und dass
das Handhabungsteil (13) ein erstes Bedienelement (25) aufweist zum Manipulieren eines mit dem ersten hülsenförmigen Element (5) der Kartuscheneinheit (4) verbundenen oder verbindbaren ersten Betätigungselements (2), und wobei das Handhabungsteil (13) ein zweites Bedienelement (20) aufweist zum Manipulieren eines mit dem zweiten hülsenförmigen Element (7) der Kartuscheneinheit (4) verbundenen oder verbindbaren zweiten Betätigungselements (3).

2. Katheter nach Anspruch 1,
wobei das Handhabungsteil (13) mit einem hohlen Führungssystem (1) verbunden oder verbindbar ist, durch dessen Innere die Betätigungselemente (2,3) geführt sind.

3. Katheter nach Anspruch 2,
wobei das Handhabungsteil (13) einen flüssigkeitsdichten Verschluss (17) aufweist zum Anflanschen des Führungssystems (1).

4. Katheter nach Anspruch 2 oder 3,
wobei das Handhabungsteil (13) flüssigkeitsdicht gegenüber der Umwelt und dem Führungssystem (1) abgedichtet ist.

5. Katheter nach einem der vorhergehenden Ansprüche,
wobei das erste Bedienelement (25) als Drückergriff ausgebildet ist.

6. Katheter nach einem der vorhergehenden Ansprüche,
wobei das zweite Bedienelement (20) als ein an einem Handgriff (13.1) des Handhabungsteils (13) angreifender Hebel ausgeführt ist, wobei über eine pumpenförmige Bewegung des Hebels das zweite Betätigungselement (3) manipulierbar ist.

7. Katheter nach einem der vorhergehenden Ansprüche,
wobei das Handhabungsteil (13) ein Rohr (28) aufweist, welches über das zweite Betätigungselement (3) mit dem zweiten hülsenförmigen Element (6) der Kartuscheneinheit (4) verbunden oder verbindbar ist.

8. Katheter nach Anspruch 7 in Kombination mit Anspruch 5, wobei das als Drückergriff ausgeführte erste Bedienelement (25) in einer gewindeförmigen Verzahnung (28.1) des Rohres (28) einrastbar ist.

9. Katheter nach einem der Ansprüche 1 bis 8,
wobei mindestens ein vorzugsweise fein justierbarer Endanschlag (29) oder mindestens eine Rasteinstellung vorgesehen ist zum Begrenzen der jeweiligen durch die Bedienelemente (20, 25) manipulierbaren Bewegung der Betätigungselemente (2, 3).

10. Katheter nach Anspruch 9,
wobei der mindestens eine Endanschlag (29) durch ein vorzugsweise als Stellschraube ausgeführtes Stellglied (31) freigebbar ist.

11. Katheter nach einem der Ansprüche 1 bis 10,
wobei das Handhabungsteil (13) mit einem hohlen Führungssystem (1) verbunden oder verbindbar ist, durch dessen Innere die Betätigungselemente (2,3) geführt sind; und
wobei das Handhabungsteil (13) einen Lueranschluss (30) aufweist zum Einführen von flüssigem Kühlmittel bzw. Pharmaka in das mit dem Handhabungsteil (13) verbundene oder verbindbare Führungssystem (1).

12. Katheter nach einem der Ansprüche 1 bis 11,
wobei das Handhabungsteil (13) mit einem hohlen Führungssystem (1) verbunden oder verbindbar ist, durch dessen Innere die Betätigungselemente (2,3) geführt sind; und
wobei das Handhabungsteil (13) ferner einen Handgriff (19) aufweist, mit welchem weitere Betätigungselemente manipulierbar sind zum gezielten Beeinflussen der Krümmung eines biegbaren Bereiches (9) des mit dem Handhabungsteil (13) verbundenen oder verbindbaren Führungssystems (1).

## Claims

1. A catheter comprising a manipulating member (13) and a cartridge unit (4) for the transvascular implantation of a self-expanding anchoring support (10) accommodated in collapsed state in the cartridge unit (4) of the catheter with a heart valve prosthesis affixed thereto, wherein the cartridge unit (4) comprises a first sleeve-shaped element (5) and a second sleeve-shaped element (7),
**characterized in that**
the cartridge unit (4) further comprises a further sleeve-shaped element (6), wherein anchoring elements (8) are formed on the further sleeve-shaped element (6) of the cartridge unit (4) into which hoops or other applicable elements formed at distal end regions of the anchoring support (10) are first engaged;
and that the manipulating member (13) comprises a first control element (25) for manipulating a first actuating element (2) connected or connectable to the first sleeve-shaped element (5) of the cartridge unit (4), and wherein the manipulating member (13) comprises a second control element (20) for manipulating a second actuating element (3) connected or connectable to the second sleeve-shaped element (7) of the cartridge unit (4).

2. The catheter according to claim 1,
wherein the manipulating member (13) is connected or connectable to a hollow guide system (1), through the interior of which the actuating elements (2, 3) are guided.

3. The catheter according to claim 2,
wherein the manipulating member (13) has a liquid-tight seal (17) for the flange-mounting of the guide system (1).

4. The catheter according to claim 2 or 3,
wherein the manipulating member (13) is liquid-impermeable vis-à-vis the environment and the guide system (1).

5. The catheter according to any one of the preceding claims,
wherein the first control element (25) is configured as a push handle.

6. The catheter according to any one of the preceding claims,
wherein the second control element (20) is configured as a lever acting on a handle (13.1) of the manipulating member (13), wherein the second actuating member (3) can be manipulated by a pumping motion of the lever.

7. The catheter according to any one of the preceding claims,
wherein the handle (13.1) comprises a tube (28) connected or connectable to the second sleeve-shaped element (6) of the cartridge unit (4) via the second actuating element (3).

8. The catheter according to claim 7 in combination with claim 5,
wherein the first control element (25) realized as a push handle can be latched into a threaded serration (28.1) of the tube (28).

9. The catheter according to any one of claims 1 to 8,
wherein at least one preferably finely adjustable limit stop (29) or at least one latching position is provided for limiting the respective movement of the actuating elements (2, 3) by the control elements (20, 25).

10. The catheter according to claim 9,
wherein the at least one limit stop (29) is releasable by an actuator (31) preferably configured as a set screw.

11. The catheter according to any one of claims 1 to 10,
wherein the manipulating member (13) is connected or connectable to a hollow guide system (1) through the interior of which the actuating elements (2, 3) are guided; and
wherein the manipulating member (13) comprises a luer connection (30) for the introducing of liquid coolant or pharmaceuticals into the guide system (1) connected or connectable to the manipulating member (13).

12. The catheter according to any one of claims 1 to 11,
wherein the manipulating member (13) is connected or connectable to a hollow guide system (1) through the interior of which the actuating elements (2, 3) are guided; and
wherein the manipulating member (13) further comprises a handle (19) which with further actuating elements can be manipulated for selectively influencing the curvature of a bendable region (9) of the guide system (1) connected or connectable to the manipulating member (13).

## Revendications

1. Cathéter comportant une partie de manutention (13) et une unité à cartouche (4) pour l'implantation transvasculaire d'un soutien d'ancrage autoexpansible (10) logé, sous forme pliée, dans l'unité à cartouche (4) du cathéter et comprenant une prothèse de valvule cardiaque fixée sur celui-ci, l'unité à cartouche comprenant un premier élément en forme de douille (5) et un second élément en forme de douille (7),
**caractérisé en ce que**
l'unité à cartouche (4) comprend en outre un autre élément en forme de douille (6), des éléments d'ancrage (8) étant réalisés sur l'autre élément en forme de douille (6) de l'unité à cartouche (4), dans lequel, des oeillets ou d'autres éléments appropriés réalisés sur des zones d'extrémité distales du soutien d'ancrage (10) sont préalablement mis en engagement ;
et **en ce que**
la partie de manutention (13) comprend un premier élément de manoeuvre (25) pour manipuler un premier élément d'actionnement (2) relié ou susceptible d'être relié au premier élément en forme de douille (5) de l'unité à cartouche (4), la partie de manutention (13) comprend un second élément de manoeuvre (20) pour manipuler un second élément d'actionnement (3) relié ou susceptible d'être relié au second élément en forme de douille (7) de l'unité à cartouche (4).

2. Cathéter selon la revendication 1,
dans lequel la partie de manutention (13) est reliée ou susceptible d'être reliée à un système de guidage creux (1) dont l'intérieur sert de guidage aux éléments d'actionnement (2, 3).

3. Cathéter selon la revendication 2,
dans lequel
la partie de manutention (13) comprend un obturateur (17) étanche aux liquides pour le bridage du système de guidage (1).

4. Cathéter selon la revendication 2 ou 3,
dans lequel la partie de manutention (13) est étanchée de façon étanche aux liquides par rapport à l'environnement et au système de guidage (1).

5. Cathéter selon l'une des revendications précédentes,
dans lequel
le premier élément de manutention (25) est réalisé sous forme de poignée-poussoir.

6. Cathéter selon l'une des revendications précédentes,
dans lequel
le second élément de manutention (20) est réalisé sous la forme d'un levier qui attaque la poignée (13.1) de la partie de manutention (13), et le second élément d'actionnement (3) est manipulable par un mouvement de pompage du levier.

7. Cathéter selon l'une des revendications précédentes,
dans lequel
la partie de manutention (13) comprend un tube (28) qui est relié ou susceptible d'être relié au second élément en forme de douille (6) de l'unité à cartouche (4) via le second élément d'actionnement (3).

8. Cathéter selon la revendication 7 en combinaison avec la revendication 5,
dans lequel
le premier élément de manutention (25) réalisé sous forme de poignée-poussoir est susceptible d'être enclenché dans une denture (28.1) en forme de pas de vis du tube (28).

9. Cathéter selon l'une des revendications 1 à 8,
dans lequel
il est prévu au moins une butée d'extrémité (29) de préférence à ajustage fin ou au moins un réglage d'enclenchement pour limiter le mouvement respectif des éléments d'actionnement (2, 3) qui est manipulable par les éléments de manoeuvre (20, 25).

10. Cathéter selon la revendication 9,
dans lequel
ladite au moins une butée d'extrémité (29) est susceptible d'être libérée par un organe de réglage (31) réalisé de préférence sous forme de vis de réglage.

11. Cathéter selon l'une des revendications 1 à 10,
dans lequel
la partie de manutention (13) est reliée ou susceptible d'être reliée à un système de guidage creux (1) dont l'intérieur sert de guidage aux éléments d'actionnement (2, 3) ; et
la partie de manutention (13) comprend un raccord de Luer pour introduire un réfrigérant ou un médicament liquide dans le système de guidage (1) relié ou susceptible d'être relié à la partie de manutention (13).

12. Cathéter selon l'une des revendications 1 à 11,
dans lequel
la partie de manutention (13) est reliée ou susceptible d'être reliée à un système de guidage creux (1) dont l'intérieur sert de guidage aux éléments d'actionnement (2, 3) ; et
la partie de manutention (13) comprend en outre une poignée à main (19) par laquelle d'autres éléments d'actionnement peuvent être manipulés pour influencer de façon ciblée la courbure d'une zone flexible (9) du système de guidage (1) relié ou susceptible d'être relié à la partie de manutention (13).
